Europäisches Patentamt

European Patent Office

Office européen des brevets

(11) Veröffentlichungsnummer: **0 214 545 B1**

(19)

(12) **EUROPÄISCHE PATENTSCHRIFT**

(45) Veröffentlichungstag der Patentschrift: **27.11.91**

(51) Int. Cl.5: **C07C 303/06, C07C 303/22**

(21) Anmeldenummer: 86111749.7

(22) Anmeldetag: 25.08.86

(54) **Verfahren zur Herstellung von 1-Aminonaphthalin-2,4,7-trisulfonsäure und 1-Aminonaphtalin-7-sulfonsäure.**

(30) Priorität: 07.09.85 DE 3531921

(43) Veröffentlichungstag der Anmeldung:
18.03.87 Patentblatt 87/12

(45) Bekanntmachung des Hinweises auf die
Patenterteilung:
27.11.91 Patentblatt 91/48

(84) Benannte Vertragsstaaten:
CH DE FR GB IT LI

(56) Entgegenhaltungen:
EP-A- 0 052 307
EP-A- 0 063 271
DE-A- 22 545
DE-A- 215 338

(HOUBEN-WEYL): "METHODEN DER ORGANI-
SCHEN CHEMIE", Band IX, Schwefel-, Selen-,
Tellurverbindungen, Auflage 4, 1955, Seiten
494,495, Georg Thieme Verlag, Stuttgart, DE

(73) Patentinhaber: **BAYER AG**

**W-5090 Leverkusen 1 Bayerwerk(DE)**

(72) Erfinder: **Streicher, Willi, Dr.**
**Andreas-Gryphius-Strasse 7**
**W-5000 Köln 80(DE)**
Erfinder: **Marzolph, Gerhard, Dr.**
**Semmelweisstrasse 87a**
**W-5000 köln 80(DE)**
Erfinder: **Behre, Horst, Dr.**
**Zur alten Linde 12**
**W-5068 Odenthal(DE)**
Erfinder: **Blank, Heinz Ulrich, Dr.**
**Am Geusfeld 35**
**W-5068 Odenthal(DE)**

**Beschreibung**

Die Erfindung betrifft ein neues Verfahren zur Herstellung von 1-Aminonaphthalin-2,4,7-trisulfonsäure und 1-Aminonaphthalin-7-sulfonsäure (Clevesäure-1,7) aus 1-Aminonaphthalin-4-sulfonsäure (p-Naphthionsäure) oder - allgemeiner - aus 1-Aminonaphthalin oder 1-Aminonaphthalin-mono- und/ oder -disulfonsäuren, deren Sulfonsäuregruppe(n) sich in 2-, 4- und/oder 7-Stellung befindet (befinden).

Clevesäure-1,7 ist ein wichtiges Zwischenprodukt für die Herstellung von Farbstoffen (siehe Ullmanns Enzyklopädie der Technischen Chemie, 4. Auflage, 1979, Band XVIII, Seiten 109 - 110).

Bislang wird Clevesäure-1,7 in einem vielstufigen Verfahren aus Naphthalin über die Naphthalin-β-sulfonsäure, die zu einem Gemisch aus 5-Nitro- und 8-Nitronaphthalin-2-sulfonsäure nitriert wird, hergestellt. Das Gemisch der beiden isomeren Nitronaphthalinsulfonsäuren wird in die beiden Isomere aufgetrennt und die beiden Isomere zu Clevesäure-1,7 bzw. Clevesäure-1,6 reduziert. Beide Clevesäuren fallen in einer Ausbeute von je 34 % der Theorie an (siehe Winnacker-Küchler, Chemische Technologie, 2. Auflage, 1959, Band 3; Organische Technologie I, Seiten 868 - 869). Die Auftrennung in die einzelnen Isomere kann jedoch auch erst auf der Stufe der Aminonaphthalinsulfonsäuren vorgenommen werden (siehe Winnacker-Küchler Technologie, 4. Auflage, 1982, Band 6; Organische Technologie II, Seite 264). Nachteilig an diesen beiden Verfahren ist die niedrige Ausbeute an Clevesäure-1,7 und der Zwangsanfall an Clevesäure-1,6.

Es hat daher nicht an Versuchen gefehlt, Verfahren aufzufinden, die Clevesäure-1,7 in besseren Aufbeuten liefern und ohne den Zwangsanfall an Clevesäure-1,6. So wird in der DE-OS 25 35 337 die Herstellung von Clevesäure-1,7 durch Ammonolyse und Desulfonierung von 1-Chlornaphthalin-4,7-disulfonsäure beschrieben. Dieses Verfahren hat jedoch den Nachteil, daß zur Herstellung der 1-Chlornaphthalin-4,7-disulfonsäure reines 1-Chlornaphthalin benötigt wird, dieses aber technisch nicht preiswert zur Verfügung steht.

In Yuki Gosei Kagaku Kyokai Shi 29 (1971) 12, 1129 (Chemical Abstract 76, 140 292 R) wird die Herstellung von Clevesäure-1,7 durch Sulfonierung von 1-Naphthol mit anschließender Hydrolyse zur 1-Naphthol-7-sulfonsäure und anschließender Bucherer-Reaktion beschrieben. Dieses Verfahren hat jedoch den Nachteil, daß es die Zwischenisolierung der 1-Naphthol-7-sulfonsäure verlangt und nur bei Verwendung wäßriger Salzsäure für die Hydrolyse zu guten Ausbeuten führt. Infolgedessen ist das Verfahren aufwendig und wirft erhebliche Korrosionsprobleme auf.

Ferner ist aus Donaldson, The Chemistry and Technology of Naphthalene Compounds, 1959, Seiten 198 und 209 bekannt, daß Clevesäure-1,7 bei der Hydrolyse von 1-Naphthylamin-2,7-disulfonsäure mit 80 %iger Schwefelsäure, oder bei der Hydrolyse von 1-Naphthylamin-2,4,7-trisulfonsäure in kochender 75 %iger Schwefelsäure, entsteht. Diese beiden Herstellungsweisen sind aber technisch uninteressant, weil für die Ausgangsverbindungen, die 1-Aminonaphthalin-2,4,7-trisulfonsäure und die 1-Aminonaphthalin-2,7-disulfonsäure, bislang noch keine wirtschaftlichen Herstellungsverfahren bekannt sind. Für die Herstellung der 1-Naphthylamin-2,4,7-trisulfonsäure wurden bislang nur folgende Verfahren vorgeschlagen:

1. Die Umsetzung von 1-Aminonaphthalin-4-sulfonsäure (Naphthionsäure) mit 3 bis 4 Teilen 40 %igem Oleum bis 120 °C (siehe Friedländer I, Seite 331: DE-PS 22 545). Es wird aber bereits in der Beschreibung des patentierten Verfahrens darauf hingewiesen, daß die Trisulfonsäure nur in unbefriedigender Ausbeute erhalten wird, weil Naphthionsäure durch Oleum in der Wärme oxidativ abgebaut wird. Außerdem wird in der Britischen Patentschrift No. 15 223 (1893) bei der Diskussion des in der Deutschen Patentschrift No. 22 545 beschriebenen Verfahrens darauf hingewiesen, daß bei der Umsetzung der Naphthionsäure mit Oleum mindestens 2 isomere Trisulfonsäuren, nämlich die 1-Aminonaphthalin-2,4,6- und die 1-Aminonaphthalin-2,4,7-trisulfonsäure entstehen.

2. Durch Umsetzung von 1-Aminonaphthalin-7-sulfonsäure (Clevesäure-1,7) oder 1-Aminonaphthalin-4,7-disulfonsäure oder deren Salze mit Oleum bei Temperaturen von 50 bis 100 °C (Britische Patentschrift No. 15 223 (1893)). Dieses Verfahren ist technisch uninteressant, weil es als Ausgangsverbindungen Clevesäure-1,7 oder 1-Aminonaphthalin-4,7-disulfonsäure erfordert, die ihrerseits nur schwer und nur über viele Herstellungs- und Reinigungsstufen (zur Abtrennung der Isomeren) zugänglich sind. Das in Friedländer I, Seite 407 (DE-PS 41 957) beschriebene Verfahren zur Herstellung von 1-Naphthylamin-4,7-disulfonsäure durch Sulfonierung von Naphthionsäure, ist für eine Herstellung dieser Disulfonsäure in technischem Maßstab ungeeignet. Die 1-Naphthylamin-4,7-disulfonsäure fällt im Gemenge mit der isomeren 1-Naphthylamin-4,6-disulfonsäure an. Eine Trennung des anfallenden Disulfonsäuregemisches über die Calciumsalze wäre zwar möglich, ist aber technisch uninteressant. Für das Verfahren sind auch keine Ausbeuten angegeben.

Überraschenderweise wurde nun gefunden, daß man 1-Amino-naphthalin-2,4,7-trisulfonsäure in ausgezeichneten Ausbeuten und praktisch isomerenfrei durch Sulfonieren von a) 1-Aminonaphthalin oder b) 1-Aminonaphthalin-mono- und/oder -disulfonsäuren, deren Sulfonsäuregruppe(n) sich in 2-, 4- und/oder 7-

EP 0 214 545 B1

Stellung befindet (befinden), vorzugsweise Naphthionsäure, erhalten kann, wenn man die Sulfonierung in Gegenwart bestimmter Zusatzstoffe vornimmt. Die Gegenwart dieser Zusatzstoffe bewirkt, daß bei der Sulfonierung des 1-Aminonaphthalins und der 1-Aminonaphthalin-mono-und/oder -disulfonsäuren, deren Sulfonsäuregruppe(n) sich in 2-, 4- und/oder 7-Stellung befindet (befinden), die neu eintretende(n) Sulfonsäuregruppe(n) selektiv noch die freie(n) 2-, 4- und/oder 7-Stellung(en) besetzt (besetzen), und daß bei der Sulfonierung praktisch kein oxidativer Abbau der 1-Aminonaphthalinsulfonsäuren erfolgt.

Diese neue Sulfonierung a) des 1-Aminonaphthalins oder b) der 1-Aminonaphthalin-mono- und/oder -disulfonsäuren, deren Sulfonsäuregruppe(n) sich in 2-, 4- und/oder 7-Stellung befindet (befinden), zu 1-Aminonaphthalin-2,4,7-trisulfonsäure eröffnet zugleich ein neues wirtschaftliches Verfahren zur Herstellung von Clevesäure-1,7 aus a) 1-Aminonaphthalin oder b) 1-Aminonaphthalin-mono- und/oder -disulfonsäuren, deren Sulfonsäuregruppe(n) sich in 2-, 4- und/oder 7-Stellung befindet (befinden), z.B. p-Naphthionsäure, denn Clevesäure-1,7 ist aus der erfindungsgemäß in technischem Maßstab herstellbaren 1-Aminonaphthalin-2,4,7-trisulfonsäure durch Hydrolyse in guten Ausbeuten erhältlich. Es wurde nämlich gefunden, daß sich die Ausbeute an Clevesäure-1,7 bei der Hydrolyse erheblich verbessern läßt, wenn man auch die Hydrolyse in Gegenwart der erfindungsgemäß für die Sulfonierungsreaktion zu verwendenden Zusatzstoffe vornimmt.

Die Erfindung betrifft daher
I. ein neues Verfahren zur Herstellung von 1-Aminonaphthalin-2,4,7-trisulfonsäure durch Sulfonierung von
    a) 1-Aminonaphthalin oder
    b) 1-Aminonaphthalin-mono- und/oder -disulfonsäuren, deren Sulfonsäuregruppe(n) sich in 2-, 4- und/oder 7-Stellung befindet (befinden), wie oben beschrieben; und
II. ein neues Verfahren zur Herstellung von Clevesäure-1,7 aus
    a) 1-Aminonaphthalin oder
    b) 1-Aminonaphthalin-mono- und/oder -disulfonsäuren, deren Sulfonsäuregruppe(n) sich in 2-, 4- und/oder 7-Stellung befindet (befinden), wonach man die nach I erhaltene 1-Aminonaphthalin-2,4,7-trisulfonsäure durch Erwärmen in wäßriger Schwefelsäure, vorzugsweise in Gegenwart der erfindungsgemäß für die Sulfonierung zu verwendenden Zusatzstoffe, zu Clevesäure-1,7 hydrolysiert.

Bei den erfindungsgemäß für die Sulfonierung zu verwendenden Zusatzstoffe handelt es sich um Säureamide oder Alkali-, Erdalkali- oder Ammonium-sulfate, -bisulfate oder Alkali-, Erdalkali- oder Ammoniumsalze solcher Säuren, die durch Schwefelsäure aus ihren Salzen verdrängt werden.

Als Säureamide kommen Carbonsäureamide, Sulfonsäureamide oder Amide der Kohlensäure in Betracht. Als Carbonsäureamide seien vor allem Formamid, Acetamid, Dimethylformamid genannt. Als Sulfonsäureamide seien vor allem Methansulfonsäureamid, Benzolsulfonsäureamid und Amidosulfonsäure genannt. Als Amide der Kohlensäure kommen vor allem Harnstoff, Alkylharnstoffe und Carbamidsäurealkyl- und -arylester in Betracht.

Als Alkali-, Erdalkali- oder Ammonium-sulfate und -bisulfate seien vor allem Natrium-, Kalium- und Ammonium-sulfat und -bisulfat genannt. Als Alkali-, Erdalkali- oder Ammonium-salze von Säuren, die durch Schwefelsäure aus ihren Salzen verdrängt werden, sind vor allem die Alkali-, Erdalkali- und Ammoniumsalze der Halogenwasserstoffsäuren, der schwefligen Säure, der Kohlensäure, der Borsäure oder der Phosphorsäure zu verstehen, ferner niederer aliphatischer Mono- und Dicarbonsäuren wie der Ameisensäure, der Essigsäure, der Propionsäure oder aber von Sulfonsäuren wie der Naphthionsäure.

Die Ammoniumionen können sich vom Ammoniak, aber auch von primären, sekundären oder tertiären Aminen ableiten oder quaternäre Ammoniumionen sein. Die Ammoniumsalze können in situ im Sulfonierungsgemisch durch Zugabe von Ammoniak oder der entsprechenden Amine zum Sulfonierungsgemisch hergestellt werden.

Gegenstand der Erfindung sind also ein
I. Verfahren zur Herstellung von 1-Aminonaphthalin-2,4,7-trisulfonsäure durch Sulfonierung von
    a) 1-Aminonaphthalin oder
    b) 1-Aminonaphthalin-mono- und/oder -disulfonsäuren, deren Sulfonsäuregruppe(n) sich in 2-, 4- und/oder 7-Stellung befindet (befinden),
dadurch gekennzeichnet, daß man die Sulfonierung zum Endprodukt mit $SO_3$ bei Temperaturen von 30 bis 120 °C in Gegenwart von - jeweils pro Mol 1-Aminonaphthalin (-mono- oder -disulfonsäure) berechnet - 0.1 bis 5 Äquivalenten Alkali-, Erdalkali- oder Ammonium-sulfaten, -bisulfaten oder Alkali-, Erdalkali- oder Ammoniumsalzen von solchen Säuren, die in Schwefelsäure aus ihren Salzen verdrängt werden, oder 0.5 bis 4 Mol Säureamiden durchführt, wobei man im Falle a) das aus 1-Aminonaphthalin zunächst mit 85 - 100 gew.-%iger Schwefelsäure bei Temperaturen von 40 bis 160 °C erhaltene Monosulfonierungsgemisch und im Falle b) die 1-Aminonaphthalinmono- und/oder -disulfonsäure einsetzt, und
II. ein Verfahren zur Herstellung von 1-Aminonaphthalin-7-sulfonsäure (Clevesäure-1,7) aus 1-Amino-

3

naphthalin oder 1-Aminonaphthalin-mono-und/oder -disulfonsäuren, deren Sulfonsäuregruppe(n) sich in 2-, 4- und/oder 7-Stellung befindet (befinden), dadurch gekennzeichnet, daß man die nach Verfahren I erhaltene 1-Aminonaphthalin-2,4,7-trisulfonsäure durch Erwärmen in wäßriger Schwefelsäure hydrolysiert.

Die erfindungsgemäß bei der Sulfonierung des 1-Aminonaphthalins und der 1-Aminonaphthalin-mono- und/oder -disulfonsäuren, deren Sulfonsäuregruppe(n) sich in 2-, 4- und/oder 7-Stellung befindet (befinden) zu verwendenden Zusatzstoffe, z.B. Natriumsulfat, sind zwar schon bei der Sulfonierung anderer Naphthalin-Verbindungen verwendet worden (siehe Houben-Weyl, Methoden der Organischen Chemie, 4. Auflage, Band IX, Seiten 494 - 495:

N.N. Woroshzow, Grundlage der Synthese von Zwischenprodukten und Farbstoffen, 1966, Seiten 48 und 63). Trotzdem ist ihre Wirkung bei der Sulfonierung von 1-Aminonaphthalin und den speziellen 1-Aminonaphthalin-mono- und/oder -disulfonsäuren überraschend. Es war in keiner Weise aus den vorbeschriebenen Reaktionen vorhersehbar, daß die Zusatzstoffe eine selektive Sulfonierung des 1-Aminonaphthalins in 4-Stellung und der noch freien 2-, 4- und 7-Stellung(en) in den 1-Aminonaphthalin-mono- und/oder -disulfonsäuren, deren Sulfonsäuregruppe(n) sich in 2-, 4- und/oder 7-Stellung befindet (befinden) bewirken könnten. Es war auch nicht vorauszusehen, daß durch die Gegenwart der erfindungsgemäß zu verwendenden Zusatzstoffe die oxidativen Abbaureaktionen bei der Sulfonierung unterdrückt und die Hydrolyse der 1-Aminonaphthalin-2,4,7-trisulfonsäure so schonend verläuft, daß die gebildete Clevesäure-1,7 unter den Hydrolysebedingungen nicht weiter zum 1-Aminonaphthalin hydrolysiert wird. Die Gegenwart der erfindungsgemäß zu verwendenden Zusatzstoffe bewirkt bei der Hydrolyse der 1-Aminonaphthalin-2,4,7-trisulfonsäure, daß die Hydrolyse so schonend verläuft, daß sie auch in technischem Maßstab vorgenommen werden kann.

Die erfindungsgemäß zu verwendenden Zusatzstoffe werden sowohl bei der Sulfonierung des 1-Aminonaphthalins als auch bei der Sulfonierung des 1-Aminonaphthalin-mono- und -disulfonsäuren und bei der Hydrolyse der 1-Aminonaphthalin-2,4,7-trisulfonsäure in einer Menge von 0,1 bis 5 Äquivalenten (bei Verwendung von Salzen), bevorzugt von 0,5 bis 3 Äquivalenten, besonders bevorzugt 1 bis 2 Äquivalenten oder von 0,5 bis 4 Mol (bei Verwendung der Säureamide), bevorzugt 0,8 bis 3 Mol, besonders bevorzugt 1 bis 2 Mol je Mol 1-Aminonaphthalin bzw. 1-Aminonaphthalinsulfonsäure, angewendet.

Für die erfindungsgemäße Sulfonierung der 1-Aminonaphthalin-mono- und/oder -disulfonsäuren, deren Sulfonsäuregruppe(n) sich in 2-, 4- und/oder 7-Stellung befindet (befinden), z.B. der Naphthionsäure, wird Schwefeltrioxid, vorzugsweise in Form von Oleum, als Sulfonierungsmittel verwendet. Das Schwefeltrioxid wird in einer Menge von 1 bis 3 Mol, vorzugsweise 1,25 bis 2,5 Mol je Mol einzuführender Sulfonsäuregruppe, angewendet. D.h. bei der Sulfonierung von Naphthionsäure, in die je Mol 2 Mol Sulfonsäuregruppen eingeführt werden müssen, werden 2 bis 6 Mol, vorzugsweise 2,5 bis 5 Mol Schwefeltrioxid eingesetzt.

Die Sulfonierung mit Schwefeltrioxid (Oleum) wird bei Temperaturen von 30 bis 130°C, bevorzugt 40 bis 120°C, besonders bevorzugt bei 50 bis 100°C, vorgenommen.

Die erfindungsgemäße Sulfonierung des 1-Naphthylamins wird zweistufig vorgenommen. In der ersten Sulfonierungsstufe wird das 1-Aminonaphthalin unter Verwendung von 85 bis 100 %iger Schwefelsäure als Sulfonierungsmittel bei Tempraturen von 40 bis 160°C, bevorzugt 60 bis 140°C, monosulfoniert. Das auf diese Weise erhaltene Monosulfonierungsgemisch, das im wesentlichen aus Naphthionsäure und einem gewissen Prozentsatz an 1- Aminonaphthalin-2-und -7-sulfonsäure, ferner geringen Anteilen an 1-Aminonaphthalin-2,4-disulfonsäure besteht, wird unmittelbar, d.h. ohne die Naphthionsäure zu isolieren, in der für die Sulfonierung der 1-Aminonaphthalin-mono- und/oder -disulfonsäuren, deren Sulfonsäuregruppe(n) sich in 2-, 4-und/oder 7-Stellung befindet (befinden) beschriebenen Weise mit Schwefeltrioxid bei Temperaturen von 30 bis 120°C weitersulfoniert.

Da sich die erfindungsgemäß für die Sulfonierung zu verwendenden Zusatzstoffe bereits in dem bei der Monosulfonierung erhaltenen Reaktionsgemisch befinden, erübrigt sich die Zugabe von Zusatzstoffen für die Weitersulfonierung.

Die als Ausgangsverbindung für die erfindungsgemäße Sulfonierung vorzugsweise einzusetzende p-Naphthionsäure kann sowohl als freie Naphthionsäure wie auch in Form ihrer Salze, z.B. ihrer Natrium-, Kalium- oder Ammoniumsalze, eingesetzt werden. Für die Sulfonierung eignet sich sowohl reine p-Naphthionsäure wie auch rohe p-Naphthionsäure. Die eingesetzten Naphthionsäuren sollten lediglich trocken sein. Anhaftende Reste an Feuchtigkeit müssen durch eine entsprechend erhöhte Oleum-Menge ausgeglichen werden. Das als Ausgangsverbindung für die erfindungsgemäße Sulfonierung einzusetzende 1-Aminonaphthalin kann in fester Form, als Schmelze oder aber auch in Form seiner Salze, beispielsweise als Naphthylammoniumhydrogensulfat, eingesetzt werden.

Die erfindungsgemäße Sulfonierung der 1-Aminonaphthalin-mono- und/oder -disulfonsäuren, deren Sulfonsäuregruppe(n) sich in 2-, 4- und/oder 7-Stellung befindet (befinden) kann auf verschiedene Weise

EP 0 214 545 B1

vorgenommen werden. Die verschiedenen Ausführungsmöglichkeiten werden nachstehend am Beispiel der Sulfonierung der p-Naphthionsäure erläutert. Die Sulfonierung kann z.B. vorgenommen werden, indem man die Suspension der p-Naphthionsäure in wasserfreier Schwefelsäure mit den vorgesehenen Zusatzstoffen versetzt, zum Gemisch anschließend langsam die vorgesehene Oleum-Menge dosiert und während der Oleum-Zugabe die Temperatur des Reaktionsgemisches stufenlos oder stufenweise von etwa 30 - 50°C auf 100 - 120°C erhöht und die Sulfonierung bei 100 bis 120°C zu Ende führt. Anstelle der mit den vorgesehenen Zusatzstoffen versetzten Suspension der p-Naphthionsäure kann man auch das bei der Monosulfonierung des 1-Aminonaphthalins anfallende, die Zusatzstoffe bereits enthaltende Monosulfonierungsgemisch einsetzen.

In einer weiteren Ausführungsform werden die vorgesehenen Mengen an Zusatzstoffen und Oleum nicht insgesamt zugegeben, sondern in mehrere Portionen aufgeteilt und diese Zusatzstoffe - und Oleum-Portionen im Wechsel der in der wasserfreien Schwefelsäure suspendierten Naphthionsäure zugegeben und nach Zugabe jeder Oleum-Portion die Temperatur des Sulfonierungsgemisches stufenweise erhöht, so daß die Anfangstemperatur der Sulfonierungsmischung von etwa 30 - 50°C nach Zugabe der letzten Oleum-Portion auf der Endtemperatur von 100 - 120°C angekommen ist. Die Sulfonierung wird bei 100 - 120°C zu Ende geführt.

Bei dieser portionsweisen Zugabe der Zusatzstoffe und Oleum wird die Menge an Zusatzstoffen jedoch vorteilhaft so aufgeteilt, daß vor Zugabe der ersten Oleum-Portion mindestens 1 Äquivalent (bei Salzen) oder 1 Mol (bei Säureamiden) Zusatzstoffe je Mol p-Naphthionsäure und/oder 1-Naphthylamin im Reaktionsgemisch vorliegt.

Als vorteilhafter hat sich jedoch erwiesen, die vorgesehenen Mengen an Zusatzstoffen und wasserfreier Schwefelsäure bei einer Temperatur von 20 bis 120°C, bevorzugt von 30 bis 110°C, besonders bevorzugt von 60 bis 100°C vorzulegen und die vorgesehenen Mengen an Oleum und Naphthionsäure bei einer Temperatur von 30 bis 130°C, bevorzugt von 40 bis 120°C, besonders bevorzugt von 50 bis 100°C simultan in 0,5 Stunden bis 24 Stunden zuzugeben.

Die Simultanzugabe kann dabei bei konstanter Temperatur erfolgen oder die Temperatur kann während der Zugabe stufenlos oder stufenweise erhöht werden.

Erfolgt die Simultanzugabe beispielsweise bei konstanter, unter der Sulfonierungstemperatur liegender Temperatur, so wird nach erfolgter Zugabe die Temperatur stufenlos oder stufenweise auf 100 - 120°C erhöht und die Sulfonierung bei 100 - 120°C zu Ende geführt. Es kann aber auch so verfahren werden, daß während der Simultandosierung die Temperatur stufenlos oder stufenweise auf 100 - 120°C erhöht wird und nach erfolgter Zugabe die Sulfonierung bei 100 - 120°C zu Ende geführt wird.

Die erfindungsgemäße Sulfonierung läßt sich auch so ausführen, daß zunächst nur die Einführung einer Sulfonsäuregruppe in die p-Naphthionsäure erfolgt, und daß die Einführung der zweiten Sulfonsäure gesondert in einer zweiten Sulfonierungsstufe erfolgt. Es wurde gefunden, daß bei Anwendung von nur 1,5 bis 2 Mol Schwefeltrioxid je Mol Naphthionsäure in Gegenwart der erfindungsgemäß zu verwendenden Zusatzstoffe, eine selektive Sulfonierung der 2-Stellung der Naphthionsäure erfolgt und auf diese Weise 1-Aminonaphthalin-2,4-disulfonsäure in guten Ausbeuten erhältlich wird. Durch Einwirkung von weiterem Schwefeltrioxid wird 1-Aminonaphthalin-2,4-disulfonsäure zur 1-Aminonaphthalin-2,4,7-trisulfonsäure sulfoniert.

Das nach Abschluß der Sulfonierung vorliegende Sulfonierungsgemisch ist eine Lösung von 1-Aminonaphthalin-2,4,7-trisulfonsäure in Schwefelsäure: sie enthält nur vernachlässigbar geringe Anteile an isomerer 1-Aminonaphthalin-2,4,6-trisulfonsäure. Die 1-Aminonaphthalin-2,4,7-trisulfonsäure kann aus dem Sulfonierungsgemisch durch Verdünnen mit Wasser und Aussalzen isoliert werden. Man kann das Sulfonierungsgemisch jedoch auch unmittelbar zur Herstellung der Clevesäure-1,7 verwenden.

Die Hydrolyse der 1-Aminonaphthalin-2,4,7-trisulfonsäure zur Clevesäure-1,7 wird in 60 bis 80 %iger, vorzugsweise 60 bis 75 %iger wäßriger Schwefelsäure bei Temperaturen von 130 - 175°C, vorzugsweise von 140 bis 170°C, insbesondere bevorzugt von 145 bis 165°C, vorgenommen. Die Hydrolysedauer beträgt etwa 2 bis 7 Stunden.

Die Menge an Zusatzstoff im Hydrolysegemisch sollte 0,1 bis 5 Äquivalente, bevorzugt 0,5 bis 3 Äquivalente, besonders bevorzugt 1 bis 2 Äquivalente, falls der Zusatzstoff ein Salz ist, oder 0,5 bis 4,0 Mol, bevorzugt 0,8 Mol bis 3 Mol, besonders bevorzugt 1 bis 2 Mol, falls der Zusatzstoff ein Säureamid ist, betragen, und zwar bezogen auf eingesetzte 1-Aminonaphthalin-2,4,7-trisulfonsäure bzw., falls das Sulfonierungsgemisch unmittelbar für die Hydrolyse eingesetzt wird, bezogen auf ursprünglich eingesetzte Naphthionsäure bzw. 1-Aminonaphthalin.

Hydrolysezeit und Hydrolysetemperatur richten sich nach der Konzentration der wäßrigen Schwefelsäure und der Menge des Zusatzstoffes im Hydrolysegemisch, und zwar in dem Sinne, daß, je geringer die Schwefelsäurekonzentration und je größer die Menge an Zusatzstoffen ist, um so länger Hydrolysezeit und um so höher die Hydrolysetemperatur sein müssen.

Bei der Hydrolyse von Sulfonierungsgemischen kann es vorteilhaft sein, falls das Sulfonierungsgemisch nur kleinere Mengen an Zusatzstoffen enthält, für die Hydrolyse weitere Mengen an Zusatzstoffen einzusetzen. Diese zusätzlichen Mengen an Zusatzstoffen können dem Sulfonierungsgemisch ober aber - vorzugsweise - dem dem Sulfonierungsgemisch für die Hydrolyse zuzusetzenden Wasser zugegeben werden.

Die Clevesäure-1,7 läßt sich aus dem Hydrolysegemisch durch Verdünnen auf eine etwa 30 - 60 %ige Schwefelsäure (Gewichtsprozent) ausfällen. Die ausgefallene Säure wird abgesaugt, mit Wasser gewaschen und getrocknet. Sie ist frei von Clevesäure-1,6.

Die Clevesäure-1,7 fällt als freie Säure an: sie kann gewünschtenfalls in bekannter Weise, z.B. durch Neutralisation ihrer wäßrigen Aufschlämmung mit entsprechenden Basen, in die gewünschten Salze überführt werden.

Für den - bevorzugten - Fall, daß man das bei der Sulfonierung von p-Naphthionsäure oder 1-Naphthylamin anfallende Sulfonierungsgemisch unmittelbar hydrolysiert, wird das Sulfonierungsgemisch einfach mit so viel Wasser vermischt, daß eine 60 bis 80 %ige Schwefelsäure entsteht. Das Vermischen des Sulfonierungsgemisches mit dem Wasser wird vorteilhaft in der Weise vorgenommen, daß man das Sulfonierungsgemisch in das Wasser einträgt und dabei die Temperatur möglichst nahe an der Siedetemperatur der Hydrolysemischung hält. Anschließend wird das Gemisch mehrere Stunden bei beispielsweise 140 bis 170 °C gerührt, und nach Beendigung der Hydrolyse mit weiterem Wasser zum Ausfällen der Clevesäure-1,7 versetzt.

Beispiel 1

In 500 g wasserfreie Schwefelsäure werden unter Rühren bei 40 - 50 °C 245 g (1 Mol) Natriumnaphthionat, danach 35,5 g (0,5 Äquivalente) Natriumsulfat eingetragen. Die Mischung wird 15 Minuten gerührt. Dann wird sie tropfenweise innerhalb von 30 Minuten mit 248 g 65 %igen Oleum (= 2 Mol $SO_3$) versetzt. Nach erneuter Zugabe von 35,5 g (0,5 Äquivalenten) Natriumsulfat wird die Sulfonierungsmischung innerhalb von 15 Minuten auf 100 °C erwärmt und bei dieser Temperatur zunächst 30 Minuten gerührt, dann mit weiteren 248 g 65 %igem Oleum tropfenweise versetzt und abermals 2 Stunden nachgerührt.

Das Sulfonierungsgemisch wird in 480 g Wasser gegossen und unter Rühren 4 Stunden bei 155 °C hydrolysiert. Dann wird das Hydrolysegemisch auf 130 °C abgekühlt und tropfenweise innerhalb 1 Stunde mit 810 g Wasser versetzt. Man läßt die Suspension unter Rühren auf Raumtemperatur abkühlen. Anschließend wird der Niederschlag abgesaugt, dreimal mit je 200 ml Wasser gewaschen, trockengepreßt und im Vakuum getrocknet.

Ausbeute: 193,7 g 87,5 %ige Clevesäure-1,7 (= 76 % d.Th.)

Gehalt an Clevesäure-1,6: <0,2 Gew.-%.

Beispiel 2

Zu 600 g wasserfreie Schwefelsäure werden 274,6 g (1 Mol) rohes Naphthionsäure-Natriumsalz (81,2 %ig), danach 132 g (2 Äquivalente) Ammoniumsulfat eingetragen und anschließend bei 50 °C 248 g 65 %iges Oleum (= 2 Mol $SO_3$) getropft. Das Sulfonierungsgemisch wird innerhalb 1 Stunde auf 100 °C erwärmt, bei dieser Temperatur zunächst 1 Stunde gerührt, dann mit weiteren 248 g 65 %igem Oleums innerhalb 1 Stunde versetzt und noch einmal 2 Stunden gerührt.

Das Sulfonierungsgemisch wird tropfenweise mit 510 g Wasser versetzt unter gleichzeitiger Steigerung der Temperatur auf 155 °C.

Nach 4-stündigem Nachrühren bei 155 °C wird das Hydrolysegemisch innerhalb 1 Stunde tropfenweise mit 830 g Wasser versetzt. Dabei fällt die Temperatur auf 120 °C. Man läßt die Suspension unter Rühren auf Raumtemperatur abkühlen. Der Niederschlag wird abgesaugt, dreimal mit je 200 ml Wasser gewaschen, gut abgepreßt und im Vakuum getrocknet.

Ausbeute: 213,2 g 78,1 %ige Clevesäure-1,7 (= 74,7 % d.Th.)

Gehalt an Clevesäure-1,6: 0 %.

Beispiel 3

In 490 g wasserfreie Schwefelsäure werden 245 g (1 Mol) Natriumnaphthionat, danach 43,5 g (0,5 Äquivalente) Kaliumsulfat eingetragen und dann 248 g 65 %iges Oleum (= 2 Mol $SO_3$) getropft. Die Sulfonierungsmischung wird auf 100 °C erwärmt und anschließend bei dieser Temperatur zunächst 30 Minuten gerührt, dann tropfenweise innerhalb von 30 Minuten mit weiteren 248 g 65 %igem Oleum versetzt und dann noch einmal 1 Stunde gerührt.

Das Sulfonierungsgemisch wird in 510 ml Wasser gegossen und 3 Stunden bei 160°C hydrolysiert. Das Hydrolysegemisch wird tropfenweise mit 850 ml Wasser versetzt. Man läßt die entstandene Suspension unter Rühren auf Raumtemperatur abkühlen. Der Niederschlag wird abgesaugt, dreimal mit je 200 ml Wasser gewaschen, gut abgepreßt und im Vakuum getrocknet.
Ausbeute: 205,5 g 80 %ige Clevesäure-1,7 (= 73,3 % d.Th.)
Gehalt an Clevesäure-1,6: 0 %.

Beispiel 4

In 600 g wasserfreie Schwefelsäure werden 223 g (1 Mol) Naphthionsäure, danach 132 g (2 Äquivalente) Ammoniumsulfat eingetragen und dann 310 g 65 %iges Oleum (= 2,5 Mol $SO_3$) getropft. Die Sulfonierungsmischung wird auf 100°C erwärmt, zunächst innerhalb 1 Stunde mit weiteren 248 g 65 %igem Oleum versetzt, danach 1 Stunde gerührt.

Das Sulfonierungsgemisch wird in 550 ml Wasser gegossen und 5 Stunden bei 150°C unter Rühren hydrolysiert. Das Hydrolysegemisch wird tropfenweise mit 820 ml Wasser versetzt. Man läßt die Suspension unter Rühren auf Raumtemperatur abkühlen. Die Niederschlag wird abgesaugt, dreimal mit je 200 ml Wasser gewaschen, gut abgepreßt und im Vakuum getrocknet.
Ausbeute: 212,1 g 78 %ige Clevesäure-1,7 (= 74,2 % d.Th.)
Gehalt an Clevesäure-1,6: 0 %.

Beispiel 5

In 588 g wasserfreie Schwefelsäure werden 242 g (1 Mol) rohe (92,2 %ige) Naphthionsäure, danach 132 g (2 Äquivalente) Ammoniumsulfat eingetragen und 186 g 65 %iges Oleum (= 1,5 Mol $SO_3$) zugegeben. Die Mischung wird auf 100°C erwärmt, bei dieser Temperatur zunächst 15 Minuten gerührt, dann tropfenweise innerhalb 1 Stunde mit weiteren 248 g 65 %igem Oleum versetzt und anschließend noch einmal 2 Stunden gerührt.

Das Sulfonierungsgemisch wird in 470 ml Wasser gegeben und 4 Stunden bei 150 bis 155°C gerührt. Das Hydrolysegemisch wird innerhalb 1 Stunde in 770 ml Wasser eingetropft. Man läßt die Suspension unter Rühren auf 40°C abkühlen. Der Niederschlag wird abgesaugt, mit Wasser gewaschen, gut abgepreßt und im Vakuum getrocknet.
Ausbeute: 210,1 g 80,2 %ige Clevesäure-1,7 (= 75,5 % d. Th.)
Gehalt an Clevesäure-1,6: 0 %.

Beispiel 6

In eine Lösung von 66 g (1 Äquivalent) Ammoniumsulfat in 441 g wasserfreier Schwefelsäure trägt man bei 40°C im Verlaufe von 2 Stunden gleichzeitig 245 g (1,0 Mol) Natriumnaphthionat und 248 g 65 %iges Oleum ein. Die Reaktionsmischung wird auf 95°C erwärmt, 1 Stunde bei dieser Temperatur gerührt, bei 95°C mit weiteren 248 g 65 %igem Oleum im Verlaufe von 1 Stunde versetzt und 1 Stunde bei dieser Temperatur nachgerührt.

Das Sulfonierungsgemisch wird in 450 ml Wasser gegossen und 4 Stunden bei 155°C hydrolysiert. Das Hydrolysegemisch wird mit 660 ml Wasser verdünnt, unter Rühren auf 50°C abgekühlt und 2 Stunden bei dieser Temperatur gerührt. Anschließend wird der Niederschlag abgesaugt, dreimal mit je 300 ml Wasser gewaschen, gut abgepreßt und im Vakuum getrocknet.

Es werden 216,1 g 91 %ige Clevesäure-1,7 (= 88,2 % der Theorie) erhalten.
Gehalt an Clevesäure-1,6: <0,05 Gew.-%.

Beispiel 7

In eine Lösung von 132 g Ammoniumsulfat (2 Äquivalente) in 490 g wasserfreier Schwefelsäure trägt man 143 g (1,0 Mol) 1-Aminonaphthalin ein. Die Mischung wird auf 100°C erwärmt und 2 Stunden bei dieser Temperatur weitergerührt. Anschließend wird auf 95°C abgekühlt und 615 g 65 %iges Oleum im Verlaufe von 4 Stunden zugetropft, danach wird 1 Stunde bei 95°C weitergerührt.

Das Sulfonierungsgemisch wird in 500 ml Wasser gegossen und 4 Stunden bei 155°C hydrolysiert. Das Hydrolysegemisch wird dann in der in Beispiel 6 beschriebenen Weise aufgearbeitet.

Es werden 166,3 g 92,4 %ige Clevesäure-1,7 (= 68,9 % der Theorie) erhalten.

Gehalt an Clevesäure-1,6: <0,05 Gew.-%.

Beispiel 8

Zur Suspension von 246,4 g (1 Mol) Natriumnaphthionat in 588 g wasserfreier Schwefelsäure werden unter Rühren und Kühlen bei 40°C 101,2 g (1 Mol) Triethylamin getropft. Das Gemisch wird bei 50°C mit 248 g 65 %igem Oleum (= 2 Mol $SO_3$) versetzt. Das Sulfonierungsgemisch wird auf 100°C erwärmt und bei dieser Temperatur zunächst 30 Minuten gerührt, dann mit weiteren 248 g 65 %igem Oleum innerhalb 1 Stunde versetzt und abermals 2 Stunden nachgerührt.

Die Sulfonierungsmischung wird in 510 ml Wasser eingegossen und durch 6-stündiges Rühren bei 155°C hydrolysiert. Das Hydrolysegemisch wird tropfenweise mit 840 ml Wasser versetzt. Man läßt die Suspension unter Rühren auf Raumtemperatur abkühlen. Der Niederschlag wird abgesaugt, mit Wasser gewaschen, abgepreßt und im Vakuum getrocknet.
Ausbeute: 227,4 g 88,7 %ige Clevesäure-1,7 (= 90,3 % der Theorie).
Gehalt an Clevesäure-1,6: 0 %.

Beispiel 9

Zur Suspension von 278,7 g (1 Mol) rohem Natriumnaphthionat (Gehalt an freier Säure: 80 %) in 600 g wasserfreier Schwefelsäure werden 18,3 g (0,25 Mol) Diethylamin getropft und anschließend 186 g 65 %iges Oleum (= 1,5 Mol $SO_3$) zugegeben. Das Gemisch wird auf 100°C erwärmt und bei dieser Temperatur zunächst 15 Minuten gerührt, dann mit weiteren 248 g 65 %igem Oleum versetzt und nach einmal 2 Stunden gerührt.

Das Sulfonierungsgemisch wird in eine Lösung von 66 g (1 Äquivalent) Ammoniumsulfat in 470 ml Wasser gegeben und 4 Stunden bei 150°C unter Rühren hydrolysiert. Das Hydrolysegemisch wird mit 750 ml Wasser versetzt. Man läßt die Suspension unter Rühren auf Raumtemperatur abkühlen. Der Niederschlag wird abgesaugt, mit Wasser gewaschen, abgepreßt und getrocknet.
Ausbeute: 218 g 71,7 %ige Clevesäure-1,7 (= 70,1 % d. Th.)
Gehalt an Clevesäure-1,6: 0,2 Gew.-%.

Beispiel 10

Die Suspension von 278,7 g (1 Mol) rohem Natriumnaphthionat in 700 g wasserfreier Schwefelsäure wird mit 39 g (0,53 Amin-Äquivalenten) eines bei der Herstellung von Propylendiamin anfallenden Abfallamins (Gehalt an N: 23 %; C: 56 %; O: 10 %; der Stickstoff liegt hauptsächlich als sekundärer und tertiärer Stickstoff vor) versetzt. Anschließend werden 186 g 65 %iges Oleum (= 1,5 Mol $SO_3$) zugegeben. Die Mischung wird auf 100°C erwärmt und bei dieser Temperatur zunächst 30 Minuten gerührt, dann mit weiteren 248 g 65 %igem Oleum versetzt und noch einmal 2 Stunden gerührt.

Das Sulfonierungsgemisch wird in 470 ml Wasser gegossen und 4 Stunden bei 155°C unter Rühren hydrolysiert. Das Hydrolysegemisch wird mit 780 ml Wasser versetzt und unter Rühren auf Raumtemperatur abgekühlt. Der Niederschlag wird abgesaugt, mit Wasser gewaschen, abgepreßt und im Vakuum getrocknet.
Ausbeute: 245 g 61 %ige Clevesäure-1,7 (= 67,4 % d.Th.)
Gehalt an Clevesäure-1,6: 0 %.

Zur Reinigung wird die rohe Clevesäure in ihr Natriumsalz überführt. Dazu wird das Rohprodukt in 300 g Wasser angeschlämmt und die Anschlämmung mit 35 %iger Natronlauge auf einen pH-Wert von 7 - 8 gestellt. Dabei wandelt sich die Suspension in eine Lösung um. Die Lösung wird 1 Stunde bei 90°C gerührt, dann abgekühlt. Der Niederschlag wird abgesaugt, mit 200 ml 10 %iger Kochsalzlösung gewaschen, abgepreßt und getrocknet.
Ausbeute: 175,6 g Clevesäure-1,7-Natiumsalz (Gehalt an freier Säure: 80 %; = 63 % d.Th.)
Gehalt an Clevesäure-1,6: 0 %.

Beispiel 11

Die Suspension von 278,7 g (1 Mol) rohem Natriumnaphthionat in 588 g wasserfreier Schwefelsäure wird mit 26,5 g (0,5 Mol) Dimethylformamid versetzt. Die Mischung wird bei 50 - 70°C mit 248 g 65 %igem Oleum (= 2 Mol $SO_3$) versetzt. Das Reaktionsgemisch wird auf 100°C erwärmt und bei dieser Temperatur zunächst 30 Minuten gerührt, dann mit weiteren 248 g 65 %igem Oleum versetzt und abschließend noch einmal 2 Stunden gerührt.

Das Sulfonierungsgemisch wird in 510 g Wasser gegossen und unter Rühren 5 Stunden bei 155°C hydrolysiert. Das Hydrolysegemisch wird tropfenweise mit 780 ml Wasser versetzt und unter Rühren auf Raumtemperatur abgekühlt. Der Niederschlag wird abgesaugt, mit Wasser gewaschen, abgepreßt und getrocknet.

Ausbeute: 214,7 g 81 %ige Clevesäure-1,7 (= 78 % d.Th.)

Gehalt an Clevesäure-1,6: 0 %.

Wurden statt der 0,5 Mol Dimethylformamid jeweils 0,5 Mol eines der nachstehend angegebenen Zusatzstoffe verwendet, so wurden die ebenfalls nachstehend angegebenen Ausbeuten an Clevesäure-1,7 erhalten:

| Beispiel Nr. | Katalysator | Ausbeute an Clevesäure-1,7 [% d.Th.] |
|---|---|---|
| 10 | Formamid | 77 |
| 11 | Harnstoff | 74 |
| 12 | Amidosulfonsäure | 70 |
| 13 | Tetraethylammoniumchlorid | 80 |
| 14 | Diethylamin | 77 |
| 15 | Triethylamin | 84 |

## Beispiel 12

Die Mischung aus 588 g wasserfreier Schwefelsäure, 278,1 g (1 Mol) rohem Natriumnaphthionat und 25,3 g (0,25 Mol) Triethylamin wird bei 50°C mit 248 g 65 %igem Oleum (= 2 Mol $SO_3$) versetzt und 1 Stunde bei 100°C gerührt. Dann wird das Gemisch mit weiteren 248 g 65 %igem Oleum versetzt und 1 Stunde bei 100°C gerührt.

Das Sulfonierungsgemisch wird in die Lösung von 66 g (1 Äquivalent) Ammoniumsulfat in 530 g Wasser gegossen und 3 Stunden bei 155°C hydrolysiert. Das Hydrolysegemisch wird tropfenweise mit 810 ml Wasser versetzt und unter Rühren auf Raumtemperatur abgekühlt. Der Niederschlag wird abgesaugt, mit Wasser gewaschen, abgepreßt und getrocknet.

Ausbeute: 207,8 g 82 %ige Clevesäure-1,7 (= 76,4 % d.Th.)

Gehalt an Clevesäure-1,6: 0 %.

## Beispiel 13

600 g wasserfreie Schwefelsäure werden mit 245 g (1 Mol) Natriumnaphthionat und 101,2 g (1 Mol) Triethylamin vermischt. Die Mischung wird bei 40°C tropfenweise mit 248 g 65 %igem Oleum (= 2 Mol $SO_3$) versetzt. Die Mischung wird innerhalb von 20 Minuten auf 100°C erhitzt und 1 Stunde bei dieser Temperatur gerührt.

Gemäß hochdruckflüssigkeitschromatographischer Analyse besteht das Sulfonierungsgemisch aus:

21,0 Gew.-% 1-Aminonaphthalin-2,4-disulfonsäure

1,8 Gew.-% Naphthionsäure

2,3 Gew.-% 1-Aminonaphthalin-2,4,7-trisulfonsäure

0,16 Gew.-% 1-Aminonaphthalin-2,4,6-trisulfonsäure

0,11 Gew.-% 1-Aminonaphthalin-4,7-disulfonsäure

Durch Zugabe von weiteren 248 g 65 %igem Oleum zum Sulfonierungsgemisch kann die Sulfonierung bis zur 1-Aminonaphthalin-2,4,7-trisulfonsäure geführt werden. Die Hydrolyse des Trisulfonierungsgemisches liefert wiederum reine Clevesäure-1,7.

Zur Isolierung der 1-Aminonaphthalin-2,4-disulfonsäure gießt man das Sulfonierungsgemisch auf ein Gemisch aus 1000 g Eis und 180 g Kochsalz und verrührt den Brei 3 Stunden bei 50°C. Man saugt den Niederschlag ab, wäscht ihn mit gesättigter Kochsalzlösung und trocknet ihn im Vakuum.
Ausbeute: 520 g 44 %ige 1-Aminonaphthalin-2,4-disulfonsäure (= 75,5 % d.Th.)

Beispiel 14 (Vergleichsbeispiel)

600 g wasserfreie Schwefelsäure werden bei 40°C mit 223 g (1 Mol) Naphthionsäure versetzt. Die Suspension wird bei 40 - 50°C mit 248 g 65 %igem Oleum (= 2 Mol $SO_3$) versetzt und anschließend auf 100°C erwärmt. Dann gibt man abermals 248 g 65 %iges Oleum zu und rührt das Sulfonierungsgemisch 2 Stunden bei 100°C nach.

Das Sulfonierungsgemisch wird in 510 ml Wasser getropft und anschließend durch 4-stündiges Erhitzen auf 155°C hydrolysiert. Das Hydrolysegemisch wird tropfenweise mit 840 ml Wasser versetzt und unter Rühren auf Raumtemperatur abgekühlt. Der Niederschlag wird abgesaugt und dreimal mit je 200 ml Wasser gewaschen.

Man erhält einen schlecht absaugbaren schwarzen Filterkuchen, der nach dem Abpressen und Trockensaugen 292 g wiegt und 30,5 Gew.-% Clevesäure-1,7 und 9,2 Gew.-% Clevesäure-1,6 enthält.
Ausbeute: Clevesäure-1,7 40 % d.Th.
Clevesäure-1,6 12,1 % d.Th.

**Patentansprüche**

1.  Verfahren zur Herstellung von 1-Aminonaphthalin-2.4.7-trisulfonsäure durch Sulfonierung von
    a) 1-Aminonaphthalin oder
    b) 1-Aminonaphthalinmono- und/oder -disulfonsäuren, deren Sulfonsäuregruppe(n) sich in 2-, 4- und/oder 7-Stellung befindet (befinden),

    dadurch gekennzeichnet, daß man die Sulfonierung zum Endprodukt mit $SO_3$ bei Temperaturen von 30 bis 120°C in Gegenwart von - jeweils pro Mol 1-Aminonaphthalin (-mono- oder disulfonsäure) berechnet - 0.1 bis 5 Äquivalenten Alkali-, Erdalkali-oder Ammonium-sulfaten, -bisulfaten oder Alkali-, Erdalkali- oder Ammoniumsalzen von solchen Säuren, die in Schwefelsäure aus ihren Salzen verdrängt werden, oder 0.5 bis 4 Mol Säureamiden durchführt, wobei man im Falle a) das aus 1-Aminonaphthalin zunächst mit 85-100 gew.-%iger Schwefelsäure bei Temperaturen von 40 bis 160°C erhaltene Monosulfonierungsgemisch und im Falle b) die 1-Aminonaphthalinmono- und/oder -disulfonsäuren einsetzt.

2.  Verfahren gemäß Anspruch 1, dadurch gekennzeichnet, daß man die Sulfonierung in Gegenwart von Alkali-, Erdalkali- oder Ammonium-sulfaten, bisulfaten, -halogeniden, -phosphaten, -boraten, -carbonaten, -bicarbonaten, -sulfiten, -bisulfiten, -acetaten oder -naphthionaten vornimmt.

3.  Verfahren zur Herstellung von 1-Aminonaphthalin-7-sulfonsäure (Clevesäure-1,7) aus 1-Aminonaphthalin oder 1-Aminonaphthalin-mono- und/oder -disulfonsäuren, deren Sulfonsäuregruppe(n) sich in 2-,4- und/oder 7-Stellung befindet (befinden), dadurch gekennzeichnet, daß man die nach Verfahren gemäß Ansprüchen 1 oder 2 erhaltene 1-Aminonaphthalin-2.4.7-trisulfonsäure durch Erwärmen in wäßriger Schwefelsäure hydrolysiert.

4.  Verfahren nach Anspruch 3, dadurch gekennzeichnet, daß man die Hydrolyse in 60-80 %iger wäßriger Schwefelsäure bei 140-170°C vornimmt und die Clevesäure-1,7 durch Verdünnen des Hydrolysegemisches auf 30-60 %ige Schwefelsäure ausfällt.

**Claims**

1.  Process for the preparation of 1-aminonaphthalene-2,4,7-trisulphonic acid by sulphonation of
    a) 1-aminonaphthalene or
    b) 1-aminonaphthalenemono- and/or -disulphonic acids, the sulphonic acid group(s) of which is (are) in the 2-, 4- and/or 7-position,
    characterised in that the sulphonation to give the end product is carried out with $SO_3$ at temperatures from 30 to 120°C in the presence of - calculated in each case per mole of 1-aminonaphthalene(-mono- or - disulphonic acid) - 0.1 to 5 equivalents of alkali metal, alkaline earth metal or ammonium sulphates or bisulphates or alkali metal, alkaline earth metal or ammonium salts of those acids which are displaced from their salts in sulphuric acid, or 0.5 to 4 mol of acid amides, in case a) the monosulphonation mixture obtained from 1-aminonaphthalene initially with 85-100% strength by weight sulphuric acid at temperatures from 40 to 160°C being used and in case b) the 1-aminonaphthalenemono- and/or -disulphonic acids being used.

2. Process according to Claim 1, characterised in that the sulphonation is carried out in the presence of alkali metal, alkaline earth metal or ammonium sulphates, bisulphates, halides, phosphates, borates, carbonates, bicarbonates, sulphites, bisulphites, acetates or naphthionates.

3. Process for the preparation of 1-aminonaphthalene-7-sulphonic acid (1,7-Cleve's acid) from 1-amino-naphthalene or 1-aminonaphthalene-mono- and/or - disulphonic acids, the sulphonic acid group(s) of which is (are) in the 2-, 4- and/or 7-position, characterised in that the 1-aminonaphthalene-2,4,7-trisulphonic acid obtained by the process of Claim 1 or 2 is hydrolysed by warming in aqueous sulphuric acid.

4. Process according to Claim 3, characterised in that the hydrolysis is carried out in 60-80% strength aqueous sulphuric acid at 140-170°C and the 1,7-Cleve's acid is precipitated by dilution of the hydrolysis mixture to 30-60% strength sulphuric acid.

**Revendications**

1. Procédé de production d'acide 1-aminonaphtalène-2,4,7-trisulfonique par sulfonation
   a) de 1-aminonaphtalène ou
   b) d'acides 1-aminonaphtalènemono- et/ou -disulfoniques dont le ou les groupes acide sulfonique se trouvent en positions 2, 4 et/ou 7,
   caractérisé en ce qu'on effectue la sulfonation jusqu'au produit final avec du SO₃ à des températures de 30 à 120°C en présence de - calculé dans chaque cas par mole de 1-aminonaphtalène ou d'acide 1-aminonaphtalène (mono-ou -disulfonique) - de 0,1 à 5 équivalents de sulfates ou bisulfates de métaux alcalins, de métaux alcalino-terreux ou d'ammonium ou de sels de métaux alcalins, de métaux alcalino-terreux ou d'ammonium d'acides qui sont déplacés de leurs sels dans l'acide sulfurique, ou de 0,5 à 4 moles d'amides d'acides, en utilisant dans le cas a) le mélange de monosulfonation obtenu tout d'abord à partir de 1-aminonaphtalène avec de l'acide sulfurique à 85-100 % en poids à des températures de 40 à 160°C et, dans le cas b), les acides 1-aminonaphtalènemono- et/ou disulfoniques.

2. Procédé suivant la revendication 1, caractérisé en ce qu'on conduit la sulfonation en présence de sulfates, bisulfates, halogénures, phosphates, borates, carbonates, bicarbonates, sulfites, bisulfites, acétates ou naphthionates de métaux alcalins, de métaux alcalino-terreux ou d'ammonium.

3. Procédé de production d'acide 1-aminonaphtalène-7-sulfonique (acide 1,7 de Cleve) à partir de 1-aminonaphtalène ou d'acides 1-aminonaphtalènemono- et/ou -disulfoniques, dont le ou les groupes acide sulfonique se trouvent en position 2, 4 et/ou 7, caractérisé en ce qu'on hydrolyse l'acide 1-aminonaphtalène 2,4,7-trisulfonique obtenu par le procédé selon les revendications 1 et 2 dans de l'acide sulfurique aqueux.

4. Procédé suivant la revendication 3, caractérisé en ce qu'on effectue l'hydrolyse dans de l'acide sulfurique aqueux à 60-80 % à 140-170°C et on précipite l'acide 1,7 de Cleve par dilution du mélange hydrolysé à une concentration en acide sulfurique de 30 à 60 %.